# EUROPEAN PATENT APPLICATION

(11) **EP 4 703 371 A1**
(43) Date of publication of application: **04.03.2026**
(21) Application number: 24198233.9
(22) Date of filing: 03.09.2024
(51) Int. Cl.: C07K 7/08, C07K 14/47

(54) **DIAGNOSTIC AND THERAPEUTIC PROPERTIES OF PEPTIDE EPITOPES DERIVED FROM BULLOUS PEMPHIGOID EXTRACELLULAR MATRIX ADHESION PROTEINS**

(71) Applicant: SeraDiaLogistics, 81545 München (DE); Gemeinnützige Salzburger Landeskliniken Betriebsgesellschaft mbH, 5020 Salzburg (AT)
(72) Inventor: The designation of the inventor has not yet been filed
(74) Representative: Vossius & Partner Patentanwälte Rechtsanwälte mbB

(57) **Abstract**

The present invention relates to a diagnostic composition comprising at least one peptide comprising an amino acid sequence selected from the group consisting of SEQ ID NO: 1 (VDELERIRRSILPYGDSMDRIEKDR), SEQ ID NO: 2 (GGAGSLGAGGAFGEA), SEQ ID NO: 3 (GPAGPPGHPGPPGPR), SEQ ID NO: 4 (EGLITLTELADSLLS), SEQ ID NO: 5 (EFQYLTGGLIEPQVH), SEQ ID NO: 6 (SSHMLTDTKTGLHFN), SEQ ID NO: 7 (FIPGPPGPPGPPGPR), SEQ ID NO: 8 (INEAIEQGTIDKALV), SEQ ID NO: 9 (AFGEAAGDRGPYGTDI) and SEQ ID NO: 10 (LITLTELADSLLSRL). Furthermore, the present invention relates to an *in vitro* method for detecting the presence or absence of a disease selected from the group of bullous pemphigoid, Parkinson's disease, and a neurological disorder comprising testing the binding of at least one peptide as identified in any of the preceding claims for binding to autoantibodies obtained from a subject suspected of suffering from one of said diseases.

## Description

The present invention relates to a diagnostic composition comprising at least one peptide comprising an amino acid sequence selected from the group consisting of SEQ ID NO: 1 (VDELERIRRSILPYGDSMDRIEKDR), SEQ ID NO: 2 (GGAGSLGAGGAFGEA), SEQ ID NO: 3 (GPAGPPGHPGPPGPR), SEQ ID NO: 4 (EGLITLTELADSLLS), SEQ ID NO: 5 (EFQYLTGGLIEPQVH), SEQ ID NO: 6 (SSHMLTDTKTGLHFN), SEQ ID NO: 7 (FIPGPPGPPGPPGPR), SEQ ID NO: 8 (INEAIEQGTIDKALV), SEQ ID NO: 9 (AFGEAAGDRGPYGTDI) and SEQ ID NO: 10 (LITLTELADSLLSRL).

Bullous pemphigoid (BP) is a rare autoimmune blistering disease characterized by large fluid-filled tense sub-epidermal blisters on the arms, legs and abdomen, affecting primarily the elderly with an annual incidence reported between 0.2 and 6 cases per 100,000 [1,2]. The high mortality of severe BP and the challenge to predict BP cases refractory to steroids and immune suppressive treatments raises the clinical urgency for biomarkers to predict refractory BP and the search for precision biologics [2,3]. The risk of BP increases among oncologic patients exposed to immune check point inhibitors [4], and type 2 diabetics undergoing treatments with dipeptidyl peptidase-4 (DDP-4) inhibitors called gliptins [5]. BP is associated with neurological disorders such as Parkinson's disease, Alzheimer's disease, dementia, and multiple sclerosis at odds ratios ranging between 2.7 and 4.5 compared to BP in elderly without neurological disorders [6-9].

The underlying dogma of BP autoimmune pathology is age-related breakdown in tolerance to two hemidesmosome autoantigens; transmembrane BP180 (BPAG2, collagen XVII) of the dermal-epidermal junction and intra-epidermal BP230 (BPAG1) [3,10]. Foxp3+ T-cells and IL-10 are reduced in perilesional biopsies of BP compared to skin lesions of psoriasis patients. The claim of T_{reg} cell deficiency is partially supported by evidence of lower percentage CD4+CD25hi+Foxp3+ T cells in peripheral blood of BP patients compared to healthy controls [11]. Although IL-7-lgE eosinophilia and metalloproteinase-9 are the likely trigger of initial pruritus with or without urticarial lesion, the expansion of autoreactive B cells and autoantibodies directed against both proteins drive the blister formation [2,3,10].

Anti-BP180 IgG1 and IgG3 autoantibodies activate complement-dependent cell membrane lysis and facilitate neutrophil destruction of the basement membrane having BP180 expression. The compelling evidence for immunodominant epitopes in the BP180 transmembrane non-collagenous domain 16A (NC16A) driving BP autoimmune pathology are the anti-NC16A titers strongly correlated to BP disease activity [12-15], the injection of polyclonal IgG against NC16A into neonatal mice inducing blisters like human BP [16] and the blockade of BP180-basement membrane damage by autoantibody Fab fragments binding to NC16A [17]. Eight percent of BP patients test negative using BP180-NC16A ELISA. Epitope mapping studies with four of these seronegative patients identified autoantibodies which bind to intracellular C-terminus of BP180 and not the NC16A domain [18].

Intact intra-epidermal BP230 protein is presumably inaccessible to immune cells and its pathogenic role remains controversial. The BP230 C-terminus structure complexed to collagen XVII (BP180) intracellular domain likely becomes exposed after the disruption of the basement membrane zone (BMZ) and keratinocyte damage [19-21]. Surprisingly, between 50 and 80 percent of BP patients express anti-BP230 autoantibodies mostly of IgG4, a weak activator of complement and without C3 and IgG deposition. BP patients having BP230 autoantibodies without BP180 autoantibodies show a mild course of disease [20-22] and in some cases a direct pathological role in mucous membrane pemphigoid [20,21].

Intramolecular epitope spreading from BP180 NC16A to the collagenous intracellular and collagenous extracellular domains of BP180 as well as inter-molecular epitope spreading from collagen XVII to BP230, was revealed in the autoantibody profile of wild-type mice immunized with NC16A domain and in recipient BP180 humanized mice that receive an adoptive transfer of spleen cells from NC16A immunized mice [10,20,23]. Evidence of epitope spreading from BP230 to Collagen XVII is described in a BP case report. The patient's initial bullous lesion localized on the thigh associated with anti-BP230 autoantibodies and no detectable Collagen XVII nor NC16A autoantibodies. Later the patient developed multiple lesions on hands and trunk with high titers of anti-NC16A and anti-collagen XVII C-terminus autoantibodies [24]. The essential role of hemidesmosome autoreactivity in disrupting the adhesive properties of cell-cell junctions in chronic diseases and aging is highlighted by the anti-BP180 and/or anti-BP230 autoantibody positivity in malignancy, most conspicuous among melanoma and non-small cell lung cancer patients treated with check point inhibitors [2,3], diabetics under peptidyl peptidase-4 inhibitors [5,21] and elderly with neurological diseases [6-9,22]. Peptide fragments of BP180 generated after proteolytic cleavage and shedding of the BP180 extracellular domain or peptide fragments of BP180 and BP230 released during inflammation may harbor cryptic epitopes not presented in the intact protein. The potential of such peptides to interact with non-desmosome autoantigens and to modulate immune pathology in the extracellular matrix (ECM) warrants attention [25]. Characterization of continuous peptide epitopes rather than known epitope targets of the intact BP180 and BP230 protein per se has value not only for diagnostic biomarkers to predict risk of autoimmunity, malignancy and neurological disease but also therapeutic applications to restore the integrity of the skin barrier [25,26].

BP diagnostics routinely relies on histological examination of perilesional tissue biopsies to visualize neutrophil, eosinophil and other cell infiltrates and to detect IgG and C3 deposits by direct immunofluorescence (DIF) staining [27]. Indirect immunofluorescence (IIF) test systems are used to confirm the presence of autoantibodies. Patient sera is incubated either on monkey esophagus or salt-split normal human skin specimens and subsequently detected as a well-defined linear pattern of IgG and/or IgA binding to the basement membrane zone (BMZ) [28,29]. Alternatively, IIF reactivity is measured on a biochip mosaic of seven distinct antigens under a single microscopic field [30]. The activity and titers of specific IgG autoantibodies in patient sera against recombinantly produced BP180 and BP230 proteins are quantified using commercial ELISA [31-35]; the BP180 (NC16A) fragment and full length BP230 of the MBL system [31,32] or BP180 (NC16A) and C-terminal BP230 fragment of the EUROIMMUN systems alongside desmogleins (Dsg) to differentiate BP from pemphigus patients [33-35]. Laminin-332 ELISA are under development for diagnosis of mucous membrane pemphigoid [36].

Several GST fusion proteins of either BP180 domains [12-15] or BP230 fragments [19-22] have been expressed and used for epitope mapping with patient sera. Monoclonal antibodies (Mabs), phage library derived Fab fragments and single chain Mabs against BP180 and BP230 epitopes are under development as diagnostic reagents or for use in therapeutic applications [17,36].

In a previous report NC16A peptide RIRRSILPYGDSMDRIEK was synthesized and conjugated with keyhole limpet hemocyanin for use in the immunization of rabbits [38] or for measuring autoantibodies in canine BP [39]. Antibody responses were successfully generated against the peptide-carrier with polyclonal IgG fraction that recognized the NC16A epitopes in immunoblotting and ELISA testing of BP180NC16A fusion protein. The immunoblots of complementary peptide alone gave no binding activity [38]. In another study, BP180 peptide fragments were randomly expressed on the surface capsid of lambda bacteriophage and bio-panned with sera of ten BP patients [15]. Fifteen BP180 epitopes of 20 to 40 amino acids were identified. Eight epitopes; amino acids 120-160, 151-170, 176-203, 266-343, 282-341, 299-354, 292-367 and 353-399 mapped to the BP180 intracellular collagenous domain (ICD) and seven epitopes; amino acids 508-541 (NC16A), 567-622, 773-798, 915-985, 1080-1107, 1331-1404 and 1354-1406 mapped to the BP180 extracellular domain (ECD). The sera reactivity was detected in crude dot blots on to nitrocellulose filter by nitro blue tetrazolium (NBT) substrate buffer. None of these peptide epitopes were validated in immunoblot, ELISA or diagnostic test platforms [15].

Prior patent publications such as US11208465B2 describe the diagnosis of blistering autoimmune diseases, such as epidermolysis bullosa acquisita, dermatitis herpetiformis and bullous pemphigoid. The polypeptides used for diagnosis are immobilized and comprise at least one antigen from laminin β-4 or a variant thereof but may also comprise at least one antigen from BP180 or BP230. The examples however demonstrate the epitope mapping and the effect of laminin β-4 derived polypeptides without further describing BP180 or BP230 epitopes. The patent focuses on autoantibodies against laminin β-4 which can be specifically detected in patients who suffer from anti-p200 pemphigoid. In a prior patent application (WO2020/072937 A1) antibodies against the non-collagenous domain 16A of BP180 and BP230 are described. Fragments of BP230 and BP180 are described further as a first domain of fusion proteins, which comprise a second domain comprising a therapeutic polypeptide or fragment thereof for use in the treatment of a disease, such as bullous pemphigoid. The examples demonstrated the positive indirect immunofluorescence staining of representative BP positive sera from BP180-NC16A and BP230-C antibodies on normal human skin and monkey esophagus substrates. The use of epitope of polypeptides, such as BPAG1 (BP180) and hBPAG2 (BP230) has also been studied for use in the prevention of skin tissue rejection (WO2013/7014247A1). The polypeptides therein comprise most preferably the extracellular domains and/or NC16A to induce a sufficient number of effector T cells and plasma cells as well as an antigen specific regulatory T-cell population that are suppressive enough to constrain effector mechanisms.

As evident from the above studies, the intact NC16A domain or intact BP180 or BP230 recombinant proteins have largely guided the development of BP immunoassays in clinical use. The prior art studies use whole protein antigens successfully for diagnosis of BP, however, the purification of these recombinant proteins and requirements of monoclonal antibody immobilization are complex and costly. The conventional thinking that accurate detection of autoantibody binding requires epitopes conserved in correctly folded structures has discouraged the use of peptides containing continuous epitopes. Thus, methods and means to provide alternatives using easier to produce but nevertheless diagnostically useful peptides are required.

Owing to the enhanced mortality of elderly BP patients and the high burden of co-morbidities associated with drug exposures and BP in neurological diseases, further analytical and clinical performance evaluation of the multi-epitope NC16A peptide as well as other BP180 and BP230 peptides is warranted. The technical problem to be solved refers to the provision of reliable and cost-sensitive alternatives to diagnostic methods and compositions known in the art.

Accordingly, the present invention relates in a first aspect to a diagnostic composition comprising at least one peptide comprising an amino acid sequence selected from the group consisting of SEQ ID NO: 1 (VDELERIRRSILPYGDSMDRIEKDR), SEQ ID NO: 2 (GGAGSLGAGGAFGEA), SEQ ID NO: 3 (GPAGPPGHPGPPGPR), SEQ ID NO: 4 (EGLITLTELADSLLS), SEQ ID NO: 5 (EFQYLTGGLIEPQVH), SEQ ID NO: 6 (SSHMLTDTKTGLHFN), SEQ ID NO: 7 (FIPGPPGPPGPPGPR), SEQ ID NO: 8 (INEAIEQGTIDKALV), SEQ ID NO: 9 (AFGEAAGDRGPYGTDI) and SEQ ID NO: 10 (LITLTELADSLLSRL).

According to the present invention, the at least one peptide contains the amino acid sequence of an epitope derived from BP180 (SEQ ID NO: 11 or 12) and/or BP230 (SEQ ID NO: 13) that gives rise to a continuous epitope suitable for interacting with autoantibodies considered responsible for the onset and/or development of bullous pemphigoid.

As used herein, the terms "BP180" and "BP230" refer to basement membrane zone adhesion proteins BP180 and BP230, respectively, which can be recognized by autoantibodies of sera of subjects suffering from a disease, such as bullous pemphigoid. BP180 is also referred to as BPAG2 or collagen VII, while BP230 is also known as BPAG1. BP180 and BP230 are components of the junctional adhesion complex called hemidesmosome. The amino acid sequences of wild-type human BP180 and BP230 are available from the NCBI database or UniProt database, respectively, under the following accession number BP180: NCBI NP_000485.3, aa 490 - 562 and aa 1190 - 1497, BP230: UniProt Q03001, aa 2318 - 2649

The term "bullous pemphigoid" (BP), as used herein, refers to an acute or chronic autoimmune skin disease associated with the formation of blisters, more appropriately the skin layers between the epidermis and dermis, known as cutaneous blisters in the space.

The term "antibody" in accordance with the present invention comprises polyclonal antibodies. The isotype of the antibody is not particularly limited, and is preferably IgG, IgM or IgA, particularly preferably IgG. As used herein in accordance with the present invention, the term "antibody" refers in particular to antibodies recognizing the epitopes of BP180 and/or BP230 which are naturally produced by the body in a subject suffering from the autoimmune disease bullous pemphigoid. Thus, antibodies against BP180 and/or BP230 are also referred to as "autoantibodies".

The term "autoantibody" generally refers to an antibody that is expressed in the body and capable of reacting with an antigenic constituent of the individual's own tissue or cells. As used herein, the antigenic constituent forms an epitope or epitope cluster, from which the amino acid sequence comprising an epitope sequence that can interact with antibodies, i.e., epitopes of BP180 or BP230 is derived.

As used herein, the term "diagnostic composition" refers to a composition suitable for the evaluation of the presence or absence of a disease or pathological condition specifically the disease or pathological composition at stake according to this invention, wherein the presence of said disease or pathological condition (positive diagnosis) is associated with the interaction of the epitope(s) comprised in the at least one peptide with autoantibodies derived from a subject to be evaluated for said disease. The term "composition", as used herein, refers to a mixture containing the peptide of the invention and contains one or more additional components. Such a component as used herein may be without limitation an additional peptide of the invention, a buffer or a leaflet to use the diagnostic composition. The peptides, buffers etc. may be contained in one or more different containers.

The term "subject", as used herein, refers to a mammal, preferably a human, wherein the human may or may not have symptoms associated with the disease or pathological condition to be evaluated for diagnosis.

As used herein, the term "epitope" refers to the portion of an antigen that interacts with a particular antibody. The interaction of the epitope with the antibody derived from the subject to be evaluated for a disease or pathological condition is associated with or assists with a positive diagnosis of said disease or pathological condition.

In general, epitopes may be continuous or discontinuous. Discontinuous epitopes are composed of residues which are located on different parts of the amino acid sequence and are only brought to proximity by folding of the protein to its native structure. Continuous epitopes are composed of residues that are in close proximity in the amino acid sequence of the peptide and thus usually do not require protein folding or a secondary structure for their recognition by an antibody. The epitopes formed by in the amino acid sequence comprised in the peptides of the diagnostic composition in accordance with the present invention are continuous epitopes.

All the epitopes are derived from the C-terminal regions of BP180 (SEQ ID NO: 12) and/or BP230 (SEQ ID NO: 13). The C-terminal region of BP180 is part of the extracellular domain of BP180 (amino acids 489-1497).

No epitope peptides comprising continuous epitopes of BP180 and/or BP230 for use in diagnosis of BP have been described in any of the above-described patent applications. This study indicates a promising approach using epitope peptides to develop a cost-effective and non-invasive alternative for in vitro diagnostics for early detection of BP having improved sensitivity than current testing platforms which rely on intact proteins.

In this invention a unique peptide array of 1452 overlapping 15-mer peptides was designed from amino acid sequences of NC16A (C-terminally to the transmembrane domain of BP180), C-terminal BP180 and C-terminal BP230. NC16A is a domain in the membrane-proximal non-collagenous region of BP180 and has been described in the prior art to comprise immunodominant antigenic sites with epitope clusters that can react with autoantibodies of BP serum samples, however only if produced as (recombinant) full-length protein [43]. Immunofluorescence intensities in the array from 13 BP patients and 2 autoimmune control patients were analyzed for specific IgG reactivity and forty-nine candidate peptides were identified. Ten candidates (SEQ ID NOs 1 to 10) have been selected by analysis of peak fluorescence intensities in microarrays using a PepSlide^{®} Analyzer (Sicasys Software GmbH), which is described in Example 1 herein below.

Out of these ten peptides, seven peptides were selected for batch synthesis with N-terminus and C-terminus linkers and biotinylated for highly efficient immobilization on to streptavidin microtiter plates. The NC16A peptide was found to contain a multi-epitope. ELISA performance evaluation with sera of 36 BP versus sera of 35 non-blistering controls gave sensitivity and specificity comparable to the BP180 commercial ELISA.

The peptide(s) of the present invention involve lower production costs in comparison to recombinant proteins which have been used in the art. For example, the purification step which is usually required for producing full-length recombinant proteins as used in prior art can be omitted in the production of simple peptides. Furthermore, in contrast to peptides, proteins known in the art require an intact native folding state, which involves specific conditions (pH, temperature, salt/buffer concentration) for handling and storing. The need for certain conditions increases complexity and hence increases costs in producing, storing and handling proteins known in the art. Thus, the diagnostic composition comprising at least one peptide of the present invention involves significantly lower costs in comparison to the proteins known in the art, while at the same time providing high specificity and sensitivity towards the antibody/antibodies of a subject.

More than one peptide may be comprised in a diagnostic composition to enhance the sensitivity towards the antibody/antibodies of a subject due to the presence of more than one epitope.

Thus, in a preferred embodiment of the first aspect, the diagnostic composition comprises two, three, four or more of said peptides.

Preferably these peptides are selected from the group consisting of SEQ ID NO: 1 (VDELERIRRSILPYGDSMDRIEKDR) SEQ ID NO: 2 (GGAGSLGAGGAFGEA), SEQ ID NO: 3 (GPAGPPGHPGPPGPR), SEQ ID NO: 4 (EGLITLTELADSLLS), SEQ ID NO: 6 (SSHMLTDTKTGLHFN), SEQ ID NO: 8 (INEAIEQGTIDKALV), and SEQ ID NO: 9 (AFGEAAGDRGPYGTDI).

It is in particular preferred in accordance with the first aspect, that the peptide is that of SEQ ID NO: 1 (VDELERIRRSILPYGDSMDRIEKDR).

The peptide of SEQ ID NO: 1 (VDELERIRRSILPYGDSMDRIEKDR) is particularly preferred since it comprises two continuous epitopes.

This bears the advantage that different autoantibodies may be detected by using just one peptide which is a cost-reducing factor.

The epitope sequences of SEQ ID NO: 1 are VDELERIRRSILPYG and ILPYGDSMDRIEKDR.

In another preferred embodiment, the composition comprises peptides of SEQ ID Nos: 1 and 2. In another preferred embodiment, the composition comprises peptides of SEQ ID Nos: 1 and 3. In another preferred embodiment, the composition comprises peptides of SEQ ID Nos: 1 and 4. In another preferred embodiment, the composition comprises peptides of SEQ ID Nos: 1 and 6. In another preferred embodiment, the composition comprises peptides of SEQ ID Nos: 1 and 8. In another preferred embodiment, the composition comprises peptides of SEQ ID Nos: 1 and 9.

In another preferred embodiment, the composition comprises peptides of SEQ ID Nos: 2 and 3. In another preferred embodiment, the composition comprises peptides of SEQ ID Nos: 2 and 4. In another preferred embodiment, the composition comprises peptides of SEQ ID Nos: 2 and 6. In another preferred embodiment, the composition comprises peptides of SEQ ID Nos: 2 and 8. In another preferred embodiment, the composition comprises peptides of SEQ ID Nos: 2 and 9.

In another preferred embodiment, the composition comprises peptides of SEQ ID Nos: 3 and 4. In another preferred embodiment, the composition comprises peptides of SEQ ID Nos: 3 and 6. In another preferred embodiment, the composition comprises peptides of SEQ ID Nos: 3 and 8. In another preferred embodiment, the composition comprises peptides of SEQ ID Nos: 3 and 9.

In another preferred embodiment, the composition comprises peptides of SEQ ID Nos: 4 and 6. In another preferred embodiment, the composition comprises peptides of SEQ ID Nos: 4 and 8. In another preferred embodiment, the composition comprises peptides of SEQ ID Nos: 4 and 9.

In another preferred embodiment, the composition comprises peptides of SEQ ID Nos: 6 and 8. In another preferred embodiment, the composition comprises peptides of SEQ ID Nos: 6 and 9.

In another preferred embodiment, the composition comprises peptides of SEQ ID Nos: 8 and 9.

In another preferred embodiment, the composition comprises peptides of SEQ ID Nos: 1, 2 and 3. In another preferred embodiment, the composition comprises peptides of SEQ ID Nos: 1, 2 and 4. In another preferred embodiment, the composition comprises peptides of SEQ ID Nos: 1, 2 and 6. In another preferred embodiment, the composition comprises peptides of SEQ ID Nos: 1, 2 and 8. In another preferred embodiment, the composition comprises peptides of SEQ ID Nos: 1, 2 and 9.

In another preferred embodiment, the composition comprises peptides of SEQ ID Nos: 1, 3 and 4. In another preferred embodiment, the composition comprises peptides of SEQ ID Nos: 1, 3 and 6. In another preferred embodiment, the composition comprises peptides of SEQ ID Nos: 1, 3 and 8. In another preferred embodiment, the composition comprises peptides of SEQ ID Nos: 1, 3 and 9.

In another preferred embodiment, the composition comprises peptides of SEQ ID Nos: 1, 4 and 6. In another preferred embodiment, the composition comprises peptides of SEQ ID Nos: 1, 4 and 8. In another preferred embodiment, the composition comprises peptides of SEQ ID Nos: 1, 4 and 9.

In another preferred embodiment, the composition comprises peptides of SEQ ID Nos: 1, 6 and 8. In another preferred embodiment, the composition comprises peptides of SEQ ID Nos: 1, 6 and 9.

In another preferred embodiment, the composition comprises peptides of SEQ ID Nos: 1, 8 and 9.

In another preferred embodiment, the composition comprises peptides of SEQ ID Nos: 2, 3 and 4. In another preferred embodiment, the composition comprises peptides of SEQ ID Nos: 2, 3 and 6. In another preferred embodiment, the composition comprises peptides of SEQ ID Nos: 2, 3 and 8. In another preferred embodiment, the composition comprises peptides of SEQ ID Nos: 2, 3 and 9.

In another preferred embodiment, the composition comprises peptides of SEQ ID Nos: 2, 4 and 6. In another preferred embodiment, the composition comprises peptides of SEQ ID Nos: 2, 4 and 8. In another preferred embodiment, the composition comprises peptides of SEQ ID Nos: 2, 4 and 9.

In another preferred embodiment, the composition comprises peptides of SEQ ID Nos: 2, 6 and 8. In another preferred embodiment, the composition comprises peptides of SEQ ID Nos: 2, 6 and 9.

In another preferred embodiment, the composition comprises peptides of SEQ ID Nos: 2, 8 and 9.

In another preferred embodiment, the composition comprises peptides of SEQ ID Nos: 3, 4 and 6, In another preferred embodiment, the composition comprises peptides of SEQ ID Nos: 3, 4 and 8. In another preferred embodiment, the composition comprises peptides of SEQ ID Nos: 3, 4 and 9.

In another preferred embodiment, the composition comprises peptides of SEQ ID Nos: 3, 6 and 8. In another preferred embodiment, the composition comprises peptides of SEQ ID Nos: 3, 6 and 9.

In another preferred embodiment, the composition comprises peptides of SEQ ID Nos: 3, 8 and 9.

In another preferred embodiment, the composition comprises peptides of SEQ ID Nos: 4, 6 and 8. In another preferred embodiment, the composition comprises peptides of SEQ ID Nos: 4, 6 and 9.

In another preferred embodiment, the composition comprises peptides of SEQ ID Nos: 4, 8 and 9.

In another preferred embodiment, the composition comprises peptides of SEQ ID Nos: 6, 8 and 9.

In another preferred embodiment, the composition comprises peptides of SEQ ID Nos: 1, 2, 3 and 4. In another preferred embodiment, the composition comprises peptides of SEQ ID Nos: 1, 2, 3 and 6. In another preferred embodiment, the composition comprises peptides of SEQ ID Nos: 1, 2, 3 and 8. In another preferred embodiment, the composition comprises peptides of SEQ ID Nos: 1, 2, 3 and 9.

In another preferred embodiment, the composition comprises peptides of SEQ ID NOs: 1, 2, 4 and 6. In another preferred embodiment, the composition comprises peptides of SEQ ID NOs: 1, 2, 4 and 8. In another preferred embodiment, the composition comprises peptides of SEQ ID NOs: 1, 2, 4 and 9.

In another preferred embodiment, the composition comprises peptides of SEQ ID NOs: 1, 2, 6 and 8. In another preferred embodiment, the composition comprises peptides of SEQ ID NOs: 1, 2, 6 and 9. In another preferred embodiment, the composition comprises peptides of SEQ ID NOs: 1, 2, 8 and 9.

In another preferred embodiment, the composition comprises peptides of SEQ ID Nos: 1, 3, 4 and 6, In another preferred embodiment, the composition comprises peptides of SEQ ID Nos: 1, 3, 4 and 8. In another preferred embodiment, the composition comprises peptides of SEQ ID Nos: 1, 3, 4 and 9.

In another preferred embodiment, the composition comprises peptides of SEQ ID Nos: 1, 3, 6 and 8. In another preferred embodiment, the composition comprises peptides of SEQ ID Nos: 1, 3, 6 and 9.

In another preferred embodiment, the composition comprises peptides of SEQ ID Nos: 1, 3, 8 and 9.

In another preferred embodiment, the composition comprises peptides of SEQ ID Nos: 1, 4, 6 and 8. In another preferred embodiment, the composition comprises peptides of SEQ ID Nos: 1, 4, 6 and 9. In another preferred embodiment, the composition comprises peptides of SEQ ID NOs: 1, 4, 8 and 9.

In another preferred embodiment, the composition comprises peptides of SEQ ID Nos: 1, 6, 8 and 9.

In another preferred embodiment, the composition comprises peptides of SEQ ID Nos: 2, 3, 4 and 6. In another preferred embodiment, the composition comprises peptides of SEQ ID Nos: 2, 3, 4 and 8. In another preferred embodiment, the composition comprises peptides of SEQ ID Nos: 2, 3, 4 and 9.

In another preferred embodiment, the composition comprises peptides of SEQ ID Nos: 2, 3, 6 and 8, In another preferred embodiment, the composition comprises peptides of SEQ ID Nos: 2, 3, 6 and 9.

In another preferred embodiment, the composition comprises peptides of SEQ ID Nos: 2, 3, 8 and 9.

In another preferred embodiment, the composition comprises peptides of SEQ ID Nos: 2, 4 and 6 and 8. In another preferred embodiment, the composition comprises peptides of SEQ ID Nos: 2, 4, 6 and 9.

In another preferred embodiment, the composition comprises peptides of SEQ ID Nos: 2, 4, 8 and 9.

In another preferred embodiment, the composition comprises peptides of SEQ ID Nos: 2, 6, 8 and 9.

In another preferred embodiment, the composition comprises peptides of SEQ ID Nos: 3, 4, 6 and 8. In another preferred embodiment, the composition comprises peptides of SEQ ID Nos: 3, 4, 6 and 9.

In another preferred embodiment, the composition comprises peptides of SEQ ID Nos: 3, 4, 8 and 9.

In another preferred embodiment, the composition comprises peptides of SEQ ID Nos: 3, 6, 8 and 9.

In another preferred embodiment, the composition comprises peptides of SEQ ID Nos: 4, 6, 8 and 9.

In another preferred embodiment, the composition comprises peptides of SEQ ID Nos: 1, 2, 3, 4 and 6. In another preferred embodiment, the composition comprises peptides of SEQ ID Nos: 1, 2, 3, 4 and 8. In another preferred embodiment, the composition comprises peptides of SEQ ID Nos: 1, 2, 3, 4 and 9.

In another preferred embodiment, the composition comprises peptides of SEQ ID NOs: 1, 2, 3, 6 and 8. In another preferred embodiment, the composition comprises peptides of SEQ ID NOs: 1, 2, 3, 6 and 9. In another preferred embodiment, the composition comprises peptides of SEQ ID NOs: 1, 2, 3, 8 and 9.

In another preferred embodiment, the composition comprises peptides of SEQ ID Nos: 1, 2, 4, 6 and 8. In another preferred embodiment, the composition comprises peptides of SEQ ID Nos: 1, 2, 4, 6 and 9. In another preferred embodiment, the composition comprises peptides of SEQ ID NOs: 1, 2, 4, 8 and 9.

In another preferred embodiment, the composition comprises peptides of SEQ ID Nos: 1, 2, 6, 8 and 9.

In another preferred embodiment, the composition comprises peptides of SEQ ID Nos: 1, 3, 4, 6 and 8. In another preferred embodiment, the composition comprises peptides of SEQ ID Nos: 1, 3, 4, 6 and 9. In another preferred embodiment, the composition comprises peptides of SEQ ID NOs: 1, 3, 4, 8 and 9.

In another preferred embodiment, the composition comprises peptides of SEQ ID Nos: 1, 3, 6, 8 and 9.

In another preferred embodiment, the composition comprises peptides of SEQ ID Nos: 1, 4, 6, 8 and 9.

In another preferred embodiment, the composition comprises peptides of SEQ ID Nos: 2, 3, 4, 6 and 8. In another preferred embodiment, the composition comprises peptides of SEQ ID Nos: 2, 3, 4, 6 and 9. In another preferred embodiment, the composition comprises peptides of SEQ ID NOs: 2, 3, 4, 8 and 9. In another preferred embodiment, the composition comprises peptides of SEQ ID NOs: 2, 3, 6, 8 and 9.

In another preferred embodiment, the composition comprises peptides of SEQ ID Nos: 2, 4, 6, 8 and 9. In another preferred embodiment, the composition comprises peptides of SEQ ID NOs: 3, 4, 6, 8 and 9.

In another preferred embodiment, the composition comprises peptides of SEQ ID Nos: 1, 2, 3, 4, 6 and 8. In another preferred embodiment, the composition comprises peptides of SEQ ID Nos: 1, 2, 3, 4, 6 and 9. In another preferred embodiment, the composition comprises peptides of SEQ ID NOs: 1, 2, 3, 4, 8 and 9. In another preferred embodiment, the composition comprises peptides of SEQ ID NOs: 1, 2, 3, 6, 8 and 9.

In another preferred embodiment, the composition comprises peptides of SEQ ID Nos: 1, 2, 4, 6, 8 and 9.

In another preferred embodiment, the composition comprises peptides of SEQ ID Nos: 1, 3, 4 6, 8 and 9.

In another preferred embodiment, the composition comprises peptides of SEQ ID NOs: 2, 3, 4, 6, 8 and 9.

In another preferred embodiment, the composition comprises peptides of SEQ ID Nos: 1, 2, 3, 4, 6, 8 and 9.

The peptides comprised in the diagnostic composition of the first aspect of the present invention may be immobilized on a surface. Immobilization may be achieved via covalent or non-covalent binding between the peptide and the surface.

The term "surface", as used herein, refers to a surface, preferably a solid surface, which may be coated with or having chemical moieties, functional groups or biomolecules. A solid surface (of a solid support) without limitation may comprise membranes of immunoblots, beads and other surfaces coated with for example, biomolecules.

The chemical moieties, functional groups or biomolecules may directly interact with the peptides. Alternatively, they may interact via a linker or spacer molecule attached to this peptide.

Thus, in another preferred embodiment of the first aspect, the peptides comprise spacer and/or linker sequences for immobilization.

The term linker sequence or spacer sequence, a used herein, refers to an entity which is not part of the amino acid sequence that encodes the peptide forming the epitope and thus is not required for its antigenic function. The linker sequence or spacer sequence is used for the interaction between the peptide, chemical moiety, functional group, or biomolecule attached to the surface. A linker sequence may also refer to a sequence that joins or links peptide bonds of two amino acid sequences or polypeptide domains that are not joined by peptide bonds in nature. Spacer and/or linker sequences are known in the art. Such spacer and/or linker sequences comprise without limitation polyethylenglycol (PEG), polysarcosine (pSar), hydrophilic polymers, zwitterionic polymers, poly(amino acid) based lipopolymers, biopolymers, poly(thioglycidyl glycerol) (PTTG), and neutral GSGSGSG linkers.

In another preferred embodiment of the first aspect of the present invention, the spacer and/or linker is a PEG-biotin linker.

Biotin linker are widely utilized in the art and well known to the skilled person. Usually, biotin is used for interaction with its high affinity partner avidin or streptavidin. In a PEG-biotin linker, as used herein, the biotin serves as the functional group for interaction with the surface in accordance with the present invention (e.g., streptavidin coated surface) while PEG is a spacer which links the biotin moiety to the peptide. The fusion to the PEG linker increases water solubility and decreases steric hindrance of biotin during binding of an avidin or streptavidin molecule due to its length.

For assays used to detect interactions between biotin and streptavidin, wherein biotin is conjugated via a linker (such as PEG) to a peptide of interest, streptavidin may be attached to a surface of a solid support, such as beads, blots or microtiter plates.

Accordingly, in another preferred embodiment of the first aspect of the present invention, the peptides are immobilized on a solid support, preferably a streptavidin coated microtiter plate.

The term "microtiter plate" also referred to herein as "microplate", or "multiwell plate" refers to arrays of discrete wells that come in standard formats (96, 384 and 1536 wells) which are used for examination of the physical, chemical or biological characteristics of a quantity of samples in parallel.

Biotinylated peptide synthesis and streptavidin microtiter plate production as described herein in accordance with the present invention involve lower costs in their production in comparison to methods known in the art.

In another preferred embodiment of the first aspect, the peptides are joined by cyclization.

As used herein, the term "cyclization" means the conversion of linear peptides to a cycle form, wherein side chain-to-side chain, head-to-tail (also backbone cyclisation), head-to-side chain or side chain-to-tail can be joined. The linking of peptides can influence the biological, physical and chemical features of the peptides, such as enhancing the thermal stability, proteolytic resistance and membrane permeability. Cyclization of peptides can be achieved by methods known in the art, such as chemical, enzyme, protein tag approaches. Chemical approaches apply without limitation direct backbone cyclisation, native chemical ligation, aldehyde-based ligations, bioorthogonal reactions or disulfide formation. Enzymatic approaches apply without limitation ubtiligase variants, sortases, asparaginyl endopeptidases, transglutaminases or non-ribosomal peptide synthetases. Protein tag approaches refer without limitation to inclusion of inteins, engineered protein domains for isopeptide bond formation.

Autoantibodies to BP180 and/or BP230 are associated with the autoimmune disease bullous pemphigoid but may also be found in sera of subjects suffering from other diseases, such as Parkinson's Disease, and/or a neurological disorder, such as Alzheimer's Disease or Multiple Sclerosis. Different epitope peptides from BP180 vs. BP230 may have a different reactivity towards autoantibodies found in the sample to be evaluated and will have a different effectivity in indicating the presence or absence of the said diseases. As mentioned above BP180 NC16 is the key pathogenic target of BP180 and BP230 autoantibodies, therefore declining of anti-NC16a titers are considered to be a strong indicator of BP remission. BP230 derived epitopes provide predictive value in neurological diseases.

Accordingly, in a further preferred embodiment of the first aspect of the present invention, the diagnostic composition is used for evaluating the presence or absence of a disease selected from the group of bullous pemphigoid, Parkinson's disease, and a neurological disorder.

As used herein, the "presence or absence of a disease" means a positive or negative diagnosis of the subject regarding the disease or diseases to be evaluated. The sample to be evaluated for the presence or absence of a disease is typically derived from the blood or other body fluids of a subject and is contacted with at least one of said peptides, wherein binding of the peptide(s) to antibodies in the sample are considered to detect a presence of the disease in comparison to a reference value.

The term "evaluating" refers to the decision or contribution to the decision whether a disease is present or absent in the subject from which the sample is derived from.

The term "contribution to the decision" refers to any information received from contacting the diagnostic composition with a sample which has been derived from a subject to be evaluated that assists for but is in itself not sufficient to determine the presence or absence of a disease. It thus contributes to the decision whether the disease is present or absent. The contribution to the decision may also comprises the comparison of said information to information received from contacting the diagnostic composition with a control sample.

In accordance with the present invention, the phrase "evaluating the present or absence of a disease selected from the group of bullous pemphigoid" refers to evaluating the BP disease group versus the group of control subjects having or suspected of having dermatitis herpetifiormis (DH), epidermylosis bullous acquisita (EBA) and non-blistering systemic autoimmune disease (AID). The binding of the peptide(s) comprising the epitope(s) to autoantibodies in the serum sample of a suspected BP subject are considered to detect the presence of the BP disease in comparison to substantially lower-binding of the peptide(s) to antibodies in the serum of one of the abovementioned "control" subjects, i.e., subjects having or suspected of having dermatitis herpetifiormis (DH), epidermylosis bullous acquisita (EBA) and non-blistering systemic autoimmune disease (AID).

"Bullous pemphigoid", has been extensively described above and as mentioned, is characterized by the presence of autoantibodies against BP180 and/or BP230.

The symptoms associated with Parkinson's disease are well known in the art to include tremors in arms, legs jaw or head, muscle stiffness, slowness in movement and impaired balance and coordination leading to falls. As is also well known, the disease is characterized by neuropathological brain changes, such as the formation of abnormal proteinaceous spherical bodies called Lewy bodies leading to motor impairment in Parkinson's disease. The alpha synuclein seed amplification assay and anti-alpha synuclein autoantibody binding in the cerebral spinal fluid (CSF) show promising diagnostic performance for early-stage Parkinson's disease [44]. This technology and other biomarker assays are undergoing evaluation in accordance with the Parkinson's Disease Markers Initiative (PPMI) study protocol (https://www.ppmi-info.org/study-design). In the absence of definitive blood and CSF biomarkers of Parkinson's disease, the presence of "Parkinson's disease" as used herein refers to a diagnosis according to clinical guidelines on motor assessment, gait disorder, unified Parkinson's disease rating scale, neuropsychiatric evaluation, PKRN gene mutation, MRI and quantification of functional dopamine neuron transport by DaTSCAN imaging [45].

The BP patients known in the Art as having symptoms of pruritic dermatitis at onset, later leading to raised tense dermal blisters, are assigned according to the profile of autoantibody binding and the extent or level of binding activity to peptides of the invention. The symptoms specific to Parkinson's disease, Alzheimer' Disease, multiple sclerosis and the symptoms associated with other neurological disorders are known in the art to involve the formation of non-BP autoantibodies and in addition may also be assigned to autoantibody binding to peptides of the invention.

The term "neurological disorders" refers to diseases and pathological conditions associated with neurons, the neuronal system, the CNS.

None limiting examples of neurological disorders are Alzheimer's Disease (AD) and Multiple Sclerosis (MS). Alzheimer's disease is well described in the art as a neurodegenerative disease characterized by symptoms such as memory loss and cognitive impairment which is associated to protein misfolding leading to the formation of amyloid plaques, neurofibrillary tangles and loss of neuronal connections in the brain. Multiple Sclerosis refers to an autoimmune disease, characterized by the damage of myelin sheath usually covering nerve cells in the brain and spinal cord, which causes disruption of the transmission of signals of the CNS. This results in symptoms like loss of sensitivity, pronounced reflexes and ataxia.

The present invention relates in a second aspect to a kit comprising at least one peptide identified in the first aspect of the invention.

The various components of the kit may be packaged into one or more containers such as one or more vials. The vials may, in addition to the components, comprise preservatives or buffers for storage. The kit may comprise instructions how to use the kit. The kit is formulated/ configured for sales purposes and is preferably configured as one package.

In a third aspect, the present invention refers to an *in vitro* method for detecting the presence or absence of a disease selected from the group of bullous pemphigoid, Parkinson's disease, and a neurological disorder comprising testing the binding of at least one peptide as identified in the first or second aspect of the present invention for binding to autoantibodies obtained from a subject suspected of suffering from one of said diseases.

In accordance with the present invention, the phrase "testing the binding" comprises detecting a signal by methods known in the art, such as immunoassays. None limiting examples of immunoassays include enzyme immunoassays (EIA), radioimmunoassays (RIA), or assays using protein/peptide microarrays.

The term "enzyme-based immunoassay" refers to immunoassays using enzymes as labels and comprises non-limiting examples such as enzyme-linked immunosorbent assay (ELISA) and enzyme multiplied immunoassay technique (EMIT).

The term "ELISA" is an immunoassay well known in the art using enzymes as labels to primarily detect antigen-antibody interactions. Different types of ELISA are comprised with the term ELISA as used herein, such as sandwich ELISA, direct ELISA, indirect ELISA, and competitive ELISA.

The steps of the ELISA performed in accordance with the present invention may involve the capture of biotinylated peptides onto streptavidin coated microtiter plates and the subsequent detection of patient (IgG) autoantibody binding to epitopes. The presence of BP is diagnosed for example by immune fluorescence testing (Euroimmun Mosaic) and ELISA cut-off > 10 U/mL BP180 (MBL Cat Nr. RG-M7612-D) and / or cut-off >10 U/mL in BP230 (MBI Cat Nr, RG-M7613-D).

The term "radioimmunoassay (RIA)" refers to an immunoassay which uses radioactive isotopes, wherein bound antigen-antibody complexes emit radioactivity that are detected by methods known in the art.

The term "assay using protein/peptide microarray" refers to an assay which requires a solid surface containing a protein array, wherein the proteins are immobilized but maintained in their native conformation with the help of a coating. The array is used to study biochemical activities of the proteins, e.g., the binding to other proteins, by contacting the array with a complex solution. Binding reactions are detected and provide information about the expression levels of the protein in the sample. The steps of such assays are known to the skilled person.

The term "signal" as used herein in conjunction with "testing the binding" refers to a "detectable signal" from a sample, a "background signal" or a "reference value" from a control sample, wherein these signals are distinguished as described as follows.

The term "detectable signal", as used herein, refers to a signal that is a direct consequence of the interaction of the epitope containing peptide and antibodies present in the sample derived from a subject to be evaluated, which can be monitored directly or indirectly. For example, the detectable signal may be monitored by physical or chemical means, such as spectroscopic, colorimetric, photochemical, biochemical, immunochemical or electro-chemical endpoint methods. Accordingly, the substrate may be, e.g., a chromogenic or fluorogenic substrate that releases a colored or fluorescent compound upon selective cleavage by the protease and the detectable signal thus can be a chromogenic or fluorescent signal but is not limited thereto.

The "detectable signal" is to be distinguished from a "background signal", also generally referred to herein as "background noise", which is considered in the evaluation of the detectable signal as a signal resulting for example from the detection of unspecific interaction of the substrate with the solid surface.

A control, for example a sample of a subject which is known to be healthy or for which the disease is known to be absent, may be treated similarly to the samples of a subject to be evaluated. The signal from the control may provide a reference value which is considered in the evaluation of a presence or absence of a disease in the subject. The skilled person may apply other means known in the art to distinguish interactions between the epitope containing peptide and antibodies present in subjects suffering from the disease (positively diagnosed subjects) from interactions between antigen and antibody naturally occurring in a control sample of a known healthy (negatively diagnosed) subject.

The term "reference value", as used herein, refers to the value or range detected from a control sample treated under identical conditions as the sample to be evaluated.

For example, the peptides of the present invention may be immobilized on a solid support via non-covalent interactions between the biotin conjugated linker on the peptide and the streptavidin coated surface of the solid support. The detection of antigen-antibody interactions is for example achieved by capture of the epitope containing peptides linked with biotin on a streptavidin coated surface. The detectable signal of a control (providing the reference value) and a sample (providing the detectable signal) are then compared to each other.

In a preferred embodiment of the third aspect, the *in vitro* method is an ELISA.

In another preferred embodiment of the third aspect, the *in vitro* method is performed using immunoblots.

The term "immunoblot(s)", as used herein, refers to a blot in which a tagged ligand, such as an antibody, is used as the molecular probe. It also refers to a product resulting from a process by which separated biomolecules are allowed to adhere to any kind of supportive membrane sheets where they bind nonspecifically and then are subsequently identified by labeling with appropriately tagged antibodies.

The term "membrane", as used herein, refers to a thin sheet of natural or synthetic material that is porous and possesses any kind of binding capacity (e.g., hydrophilic, hydrophobic, or has a capacity to bind to positively or negatively charged molecules) and retains biomolecules above a certain molecular size (usually ≧3 nm). Most commonly used membranes are produced from nitrocellulose, nylon, or polyvinylidene difluoride (PVDF).

In another preferred embodiment of the third aspect, the *in vitro* method is performed using bead technology, optionally Luminex.

As used herein, the term "bead technology" refers to the means of recognizing target analytes with analyte-specific antibody coated beads. For example, the Luminex technology is a specific bead technology, wherein a mixture of beads is analyzed, wherein the beads are color-coded, precoated with the analyte specific antibody before being mixed with a sample containing analytes of interest. The analytes may then be bound by biotinylated analyte-specific detection antibodies in an antibody-antigen sandwich. Phycoerythrin (PE)-conjugated streptavidin is added and binds to the biotinylated detection antibodies. The beads are read on a dual-laser flow-based detection instrument, such as the Luminex 200^{™} or FlexMap^{®} analyzer. One laser classifies the color-coded bead and determines the analyte that is being detected. The second laser determines the magnitude of the PE-derived signal, which is in direct proportion to the amount of analyte bound.

In a fourth aspect, the present invention refers to a pharmaceutical composition comprising at least one peptide as characterized in any of the preceding embodiments.

The term "pharmaceutical composition" as used herein, refers to a mixture of a component comprising the peptide(s) as identified in any of the preceding aspects of the invention and one or more pharmaceutically acceptable carriers, excipients or adjuvants. A "pharmaceutical composition" further refers to a composition for administration to a patient, preferably a human patient. The pharmaceutical composition may be in solid, liquid or gaseous form and may be, inter alia, in the form of (a) powder(s), (a) tablet(s), (a) solution(s) or (an) aerosol(s). The route of administration may be, for example, orally, parenterally, such as subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.5 mg to 50 mg and most preferably 0.5 mg to 10 mg per day.

The term "pharmaceutically acceptable carrier" refers to a carrier that can be administered to a subject with the pharmaceutical composition of the present invention when administered in a dose sufficient to deliver a therapeutic amount of the compound, without destroying its pharmacological activity and without being toxic to the recipient.

The term "excipient", as used herein, refers to an inert constituent of a pharmaceutical composition, which imparts a beneficial physical property to a formulation such as increased protein stability and/or decreased viscosity. An "excipient" can be used as a diluent, vehicle, preservative, binder or stabilizing agent for drugs.

As used herein, the term "adjuvant" refers to an immunological agent, that modifies the effect of an immunogen. In other terms, the term "adjuvant" refers to a substance or material that when administered together or in conjunction with an antigen increases the immune response to that antigen.

In accordance with the present invention the "pharmaceutically acceptable carrier", "pharmaceutically acceptable excipient" or "pharmaceutically acceptable adjuvant" refers to a non-toxic solid, semisolid or liquid filler, diluent, encapsulating material or formulation auxiliary of any type (see also Handbook of Pharmaceutical Excipients 6 ed. 2010, Published by the Pharmaceutical Press). Examples of suitable pharmaceutical carriers, excipients and adjuvants are well known in the art and include phosphate buffered saline solutions, water, emulsions, such as oil/water emulsions, various types of wetting agents, sterile solutions, organic solvents including DMSO etc. Compositions comprising such carriers, excipients or adjuvants can be formulated by well-known conventional methods.

The pharmceutical composition of the present invention may include a peptide sequence genetically engineered into the signalling receptor of an immune cell (i.e. CD3 T-lymphocyte or natural killer cell) in order to target autoreactive CD19 B cells in the circulation and deplete the source of pathogenic autoantibody production. Alternatively, the pharmaceutical composition may allow delivery of the peptide(s) per se into the skin of BP subject to cause a blockade of the autoantibody cytotoxicity or delivery into the tissues or extracellular matrix of other disease subjects to alleviate symptoms that associate to the peptide binding.

In a fifth aspect, the present invention refers to the at least one peptide as identified in any of the preceding aspects for use in the treatment of a disease selected from the group of bullous pemphigoid, Parkinson's disease, and a neurological disorder.

As used herein, the term "treatment" refers to the reversal, alleviation or inhibition the progress of a disease, or one or more symptoms of such disease. The term "treatment" also means improvement, enhancement, amelioration the conditions of a subject suffering from a disease, preferably a human subject.

In an embodiment of the fifth aspect, the present invention refers to a method of treating a disease selected from the group of bullous pemphigoid, Parkinson's disease, and a neurological disorder, comprising administering the at least one peptide as identified in any of the preceding aspects or the pharmaceutical composition of the fourth aspect to a patient in need thereof.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The Figures show:

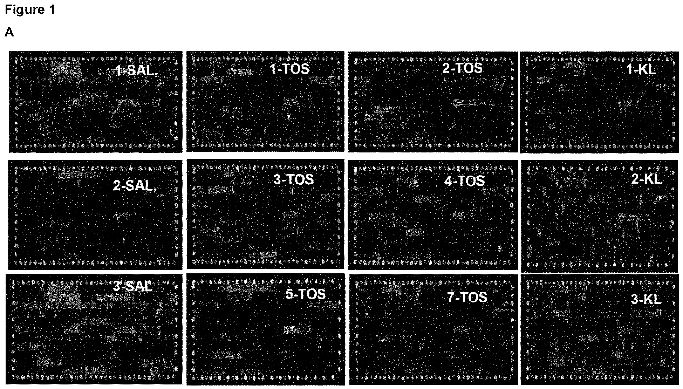

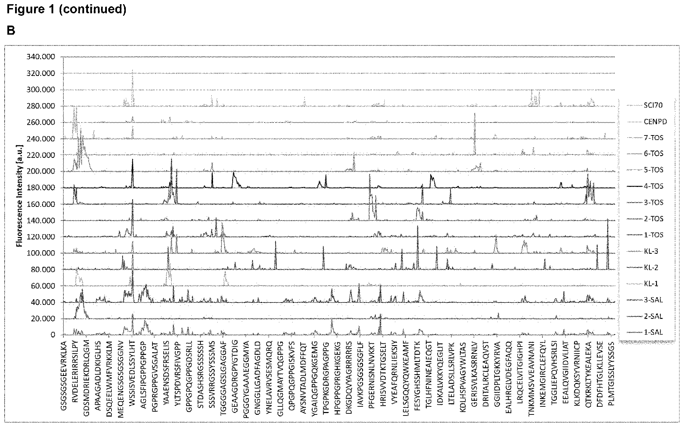

**Fig. 1****. Peptide array fluorescent Intensity map generated by patient sera reactivities.** 15-mer peptides with 14 amino acid overlap derived from sequences of BP180 NC16a, BP180 C-terminus, BP230 C-terminus indicated in Table 1 were printed in duplicates on to PepPerPrint microarray. Thirteen BP patients' site 1 (1-TOS, 2-TOS, 3-TOS, 4-TOS, 5-TOS, 6-TOS, 7-TOS, KL1, KL2, KL3, 1-SAL, 2-SAL, 3-SAL versus two non-blistering autoimmune disease sera (Scl70 and CENPB). A. Fluorescence reactivities of the human IgG (red). Internal positive controls fluorescence signals are the wells containing viral peptides circumscribing the periphery of the array, alternating anti-influenza (red) and anti-polio (green). B. Semi quantification of the relative fluorescence intensities of each serum corresponding to putative peptide epitopes.

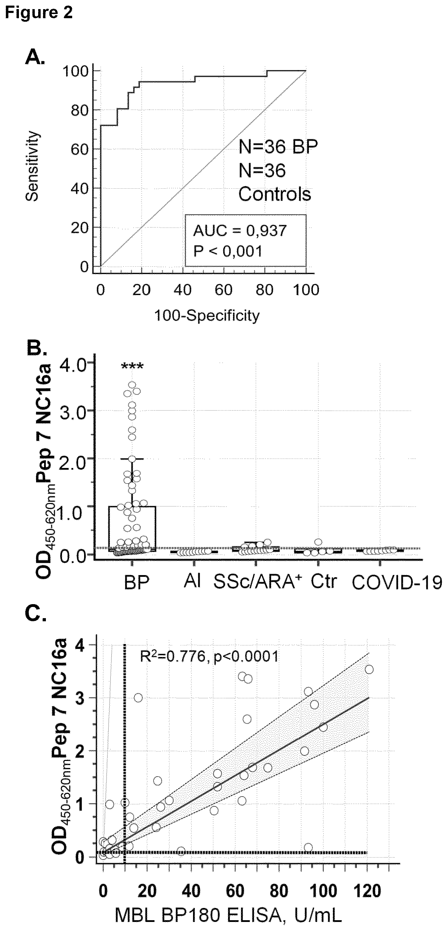

**Fig. 2****. Diagnostic performance of BP180 NC16 peptide 7 Elisa Site 1.** The biotinylated 23-mer peptide VDELERIRRSILPYGDSMDRIEKDR (BP180 aa 499 - 522, NCBI Reference Sequence: NP_000485.3) is captured on Streptavidin microtiter plates (Greiner Bio-one) and human serum is tested at 1:500 dilution. A. The Receiver Operator Curve (ROC) analysis from sera of N=36 BP confirmed in accordance with clinical examination of blister morphology, basement membrane histology and immune fluorescence positivity (Table 1) versus N=36 controls without blistering disease from Dermatology site 1. 81% sensitivity and 92 specificity, positive likelihood ratio (+LR)=9.9, negative likelihood ratio (-LR)=0.21. **B**. Distribution of IgG ELISA reactivities. Horizontal dotted line indicates cut-off >0.168 OD450-620nm. *** p<0.0001 unpaired t-test mean difference between OD values of BP versus OD values of controls. C. Method Comparison of the BP180 ELISA versus biotinylated Peptide 7-streptavidin ELISA. Passing-Bablok regression analysis Spearman Rank R2=0.776, p<0.0001. Vertical and horizontal lines indicate cut of the BP180 antigen ELISA activity and the peptide 7 Elisa reactivity respectively.

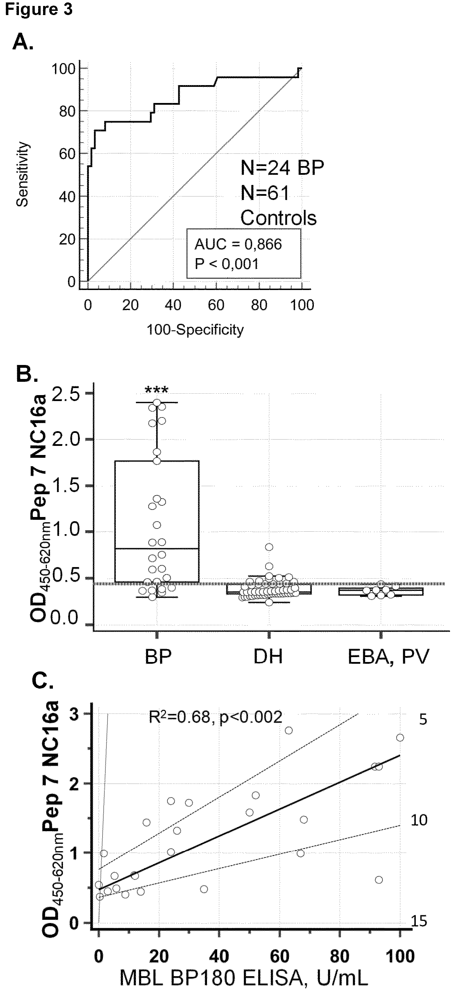

**Fig. 3****. Diagnostic performance of BP180 NC16 peptide 7 Elisa Site 2.** The biotinylated 23-mer peptide VDELERIRRSILPYGDSMDRIEKDR (BP180 aa 499 - 522, NCBI Reference Sequence: NP_000485.3) is captured on Streptavidin microtiter plates (Biomat) and human serum is tested at 1:500 dilution. **A**. The Receiver Operator Curve (ROC) analysis from sera of N=24 BP confirmed in accordance with clinical examination of blister morphology, basement membrane histology and immune fluorescence positivity versus N=61 controls with blistering diseases from Dermatology site 2. 75% sensitivity and 92 specificity, positive likelihood ratio (+LR)=9.15, negative likelihood ratio (-LR)=0.27. **B**. Distribution of IgG ELISA reactivities of patients described in Table 1. Horizontal dotted line indicates cut-off >0.49 OD _{450-620nm.} *** p<0.0001 unpaired t-test mean difference between OD values of BP versus OD values of controls. **C**. Method Comparison of the BP180 ELISA versus biotinylated Peptide 7-streptavidin ELISA. Passing- Bablok regression analysis Spearman Rank R²=0.68, p<0.002. Vertical and horizontal lines indicate cut of the BP180 antigen ELISA activity and the peptide 7 Elisa reactivity respectively.

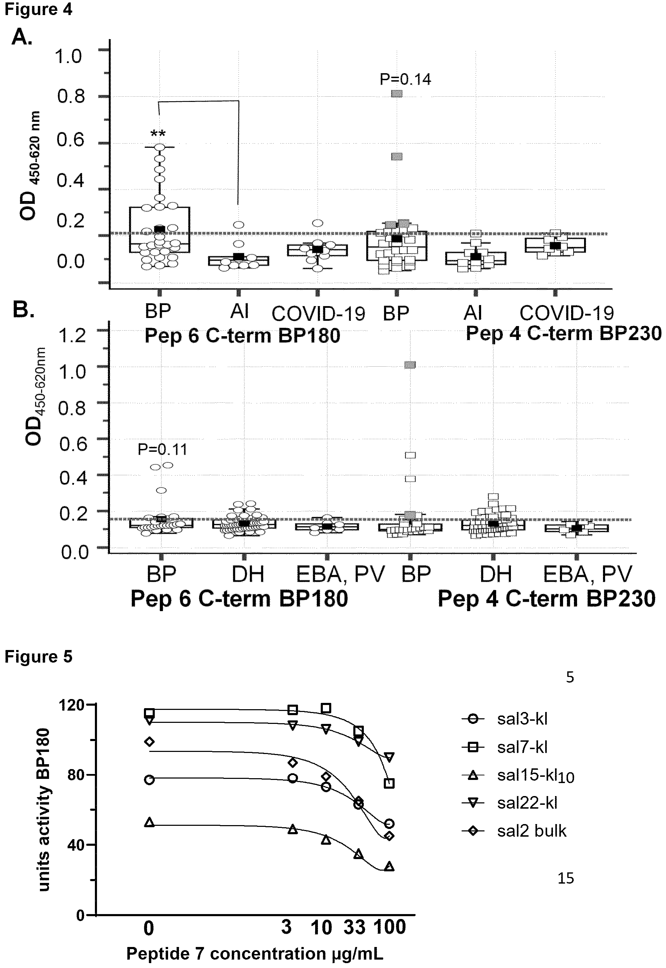

**Fig. 4****. Diagnostic performance of peptide ELISA of C-terminal BP180 and BP230.** The biotinylated C-terminus BP180 peptide AFGEAAGDRGPYGTDI (BP180 aa 1326 - 1342 from NCBI Reference Sequence: NP_000485.3) and biotinylated C-terminal BP230 SSHMLTDTKTGLHFN BP230 (aa 2389 - 2402, UniProt ID: 003001). The distributions of sera reactivity **A**. site 1 and **B**. site 2. Horizontal dotted lines indicate the cut-off >0.21 site 1 and >0.15 site 2. **p<0.02 independent student t-test. Filled symbols indicate BP patients that test positive in Pep4 C-term BP230 and negative in pep7 BP180 NC16a.
**Fig. 5****. BP180 peptide 7 inhibition of autoantibody binding activity in BP180 antigen ELISA.** Sera of five BP patients having positive autoantibodies were incubated 1 hour at room temperature with peptide 7 at 0, 3, 10, 33 and 100 µg/mL and then assessed for binding activity in the MBL BP180 commercial ELISA. Curve fit Graph pad Prism software.

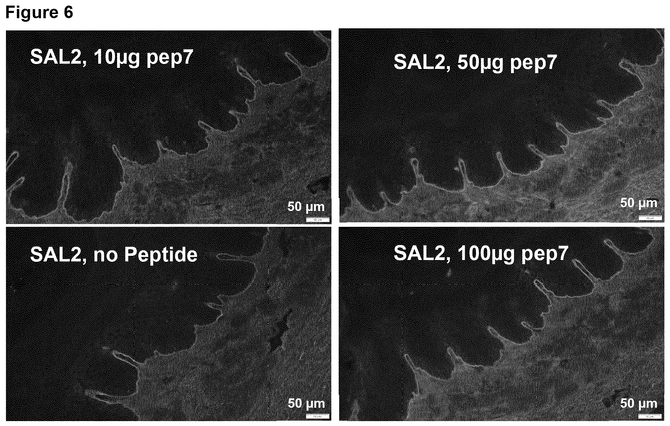

**Fig. 6****. Peptide 7 uptake and binding in epidermal basement membrane.** Indirect Immune fluorescence on monkey esophagus tissue preparations (INVOVA Diagnostics) after 1 hour room temperature incubation of BP patient serum SAL2 with 0, 10, 50 and 100µg/mL of BP180 peptide 7. Scale: 50 µm.

The invention is illustrated by the examples.

### Example 1

### Sequences

SEQ ID NO: 1 to 10 are ten identified peptides, respectively, containing epitopes of interest based on the criteria of common reactivity by different BP serum and having highest relative intensity of their fluorescence signals.
SEQ ID NO: 1: VDELERIRRSILPYGDSMDRIEKDR,
SEQ ID NO: 2 GGAGSLGAGGAFGEA,
SEQ ID NO: 3 GPAGPPGHPGPPGPR,
SEQ ID NO: 4 EGLITLTELADSLLS,
SEQ ID NO: 5 EFQYLTGGLIEPQVH,
SEQ ID NO: 6 SSHMLTDTKTGLHFN,
SEQ ID NO: 7 FIPGPPGPPGPPGPR,
SEQ ID NO: 8 INEAIEQGTIDKALV,
SEQ ID NO: 9 AFGEAAGDRGPYGTDI
SEQ ID NO: 10 LITLTELADSLLSRL

**Table 1. Derivation of Peptide sequences in peptide array**

| **Protein Origin** | **Amino acid position** | **Peptide Sequence** |
|---|---|---|
| BP180 NC16 | 491-562 | EVRKLKARVD EVRKLKARVD PYGDSMDRIE KDRLQGMAPA AGADLDKIGL HSDSQEELWM FVRKKLMMEQ EN |
| SEQ ID NO: 11 | | |
| BP180 C-terminus | 1191-1497 | NVWSSISVED LSSYLHTAGL SFIPGPPGPP GPPGPRGPPG VSGALATYAA ENSDSFRSEL ISYLTSPDVR SFIVGPPGPP GPQGPPGDSR LLSTDASHSR GSSSSSHSSS VRRGSSYSSS MSTGGGGAGS LGAGGAFGEA AGDRGPYGTD IGPGGGYGAA AEGGMYAGNG GLLGADFAGD LDYNELAVRV SESMQRQGLL QGMAYTVQGP PGQPGPQGPP GISKVFSAYS NVTADLMDFF QTYGAIQGPP GQKGEMGTPG PKGDRGPAGP PGHPGPPGPR GHKGEKGDKG DQVYAGRRRR RSIAVKP |
| SEQ ID NO: 12 | | |
| BP230 C-terminus | 2329-2649 | FL FPFGERNISN LNVKKTHRIS VVDTKTGSEL TVYEAFQRNL IEKSIYLELS GQQYQWKEAM FFESYGHSSH MLTDTKTGLH FNINEAIEQG TIDKALVKKY QEGLITLTEL ADSLLSRLVP KKDLHSPVAG YWLTASGERI SVLKASRRNL VDRITALRCL EAQVSTGGII DPLTGKKYRV AEALHRGLVD EGFAQQLRQC ELVITGIGHP ITNKMMSVVE AVNANIINKE MGIRCLEFQY LTGGLIEPQV HSRLSIEEAL QVGIIDVLIA TKLKDQKSYV RNIICPQTKR KLTYKEALEK ADFDFHTGLK LLEVSEPLMT GISSLYYSS |
| SEQ ID NO: 13 | | |

### Materials and Methods

### Human serum:

Specimens of human serum were obtained by whole blood collections in clot vacutainer tubes from clinically well-defined bullous pemphigoid (BP) and controls at two independent sites. Site 1 prospective sampling Salzburg, Austria EK Nr: 1028/2022 BP and non-blistering autoimmune diseases (Al); systemic scleroisis (SSc), anca vasculitis (ANCA) and RT-PCR positive SARS-CoV-2 coronavirus infectious disease (COVID-19); and site 2 retrospective biobank Duke Health, USA DUHS Pro00048083-AMD-8; BP and control autoimmune blistering diseases; dermatitits herpitiformis (DH), Epidermoysis accquista (EBA) and pemphigus vulgaris (PV).

### Peptides: 726 different 15-mer peptides from amino acid sequences of NC16A, C-terminal BP180 and C-terminal BP230 were constructed by solid phase peptide synthesis (SPPS) and laser-printed in duplicates on to PEPperChip Custom Peptide Microarray. The synthesis of high-density array involves high precision coupling of pre-activated and fluorenylmethoxycarbonyl protected (Fmoc) amino acids on to the microarray support in a unique microparticle toner and subsequent cycles of washing and de-protection. Selected peptides were synthesized in bulk at 1mg/mL concentrations with C-terminal PEG-biotin linker.

### Microarray Content:

PEPperCHIP^{®} Custom Peptide Microarray. Segments of BP180 (NCBI NP_000485.3, aa 490 - 562 and aa 1190 -1497) and BP230 (UniProt Q03001, aa 2318 - 2649) were linked and elongated with neutral GSGSGSG linkers at the C- and N-termini to avoid truncated peptides. The linked and elongated antigen sequences were converted into linear 15 amino acid peptides with a peptide-peptide overlap of 14 amino acids. The resulting BP180 & BP230 peptide microarrays contained 726 different peptides printed in duplicate (1,452 spots) and were framed by additional HA (YPYDVPDYAG, 46 spots) and polio (KEVPALTAVETGAT, 44 spots) control peptides.

### Samples:

Human serum samples 1-TOS, 2-TOS, 3-TOS, 4-TOS, 5-TOS, 6-TOS, 7-TOS, 1-SAL, 2-SAL, 3-SAL, KL-1, KL-2, KL-3, SCI70 and CENPD
Washing Buffer: PBS, pH 7.4 with 0.05% Tween 20; washing for 3 x 10 sec after each incubation step
Blocking Buffer: Rockland blocking buffer MB-070; for 30 min before first assay
Incubation Buffer: Washing buffer with 10% blocking buffer

### Assay Conditions:

Serum dilution of 1:500 in incubation buffer; incubation for 16 h at 4°C and orbital shaking at 140 rpm.

### Secondary Antibody:

Goat anti-human IgG (Fc) DyLight680 (0.1 µg/ml); 45 min staining in incubation buffer at RT.

### Control Antibody:

Mouse monoclonal anti-HA (12CA5) DyLight800 (0.2 µg/ml); 45 min staining in incubation buffer at RT.

### Scanner:

Innopsys InnoScan 710-IR Microarray Scanner; scanning resolution of 20 µm; scanning gain of 50 at low laser power (680 nm, red) and 10 at high laser power (800 nm, green)

Microarray Identifier: 003412_01 - 003412_04 (5 array copies for one-by-one assays)

### Peptide Elisa:

Coating Buffer: MB070S, Rockland Lot 38323
Commercial Sample Buffer: T.R.A. Medizym, 3505 Lot 147665
Commercial Wash Buffer: 10X WashB Medizym Lot 147003, dilute 1:10 in deionized water
Microtiter plates: Streptavidin-coated 96 WELL, transparent flat bottom. Greiner bio-one GmbH,
Germany (655990) or Biomat, Italy (MT0STF4-SA5/200)
Peptide 7 BP180, Peptide 6 BP180, Peptide 4 BP230, PepPerPrint synthesis
Peptide 7 17.7.2023, 10 mg/mL, Peptide 6, 1mg/mL Peptide 4, 1 mg/mL
Anti-human IgG use at 1:15:000 dilution (cat nr 609-4303Rockland)

| **Step** | **Description** |
|---|---|
| 1 | Coat peptide 2 µg/well in 250 µL coating buffer, at 4_{°}C storage > 2 days up to 2 months |
| 2 | wash 4x 290µL wash buffer |
| 3 | Prepare clinical specimens (human serum) at 1:500 or 1:1000 dilutions in Medizym T.R.A. sample buffer |
| 4 | Add 100µL per well |
| 5 | Incubate 1 hour room temperature |
| 6 | Wash 4x 290µL wash buffer |
| 7 | Add secondary antibody 1:10000 in wash buffer |
| 8 | Wash 3x 290 µL wash buffer |
| 9 | Add 100µL TMB substrate |
| 10 | Add 100µL Stop solution |
| 11 | Measure OD450nm and OD620nm reference Multiskan 96-well reader (Lot 357-706872, Thermofischer Scientific Life Technologies, Darmstadt, Germany). |

### Example 2

Tables 2 and 3 list the human serum samples obtained by ethics committee investigational review board (EC-IRB) approval at two independent clinical dermatology sites.

**Table 2. Patient characteristics. Site 1. BP= bullous pemphigoid, ANCA; N=4 anti-neutrophil autoantibodies cytoplasmic (MPO) or perinuclear (PR3), SSc; systemic sclerosis patients N=5 centromere or Sc70 positive autoantibodies, SSc ARA+= systemic sclerosis patients anti-RNA polymerase (ARA) autoantibody positive with malignancies, Geriatric; N=4 without skin disease including one 86-year male stage 2/3 Parkinson's disease (PD), Covid-19: SARS CoV-2 RT-PCR positive within 2 weeks after onset of symptoms.**

| **Table 2** | **BP site 1** | **Disease Controls site 1** | | | |
|---|---|---|---|---|---|
| | | Al (ANCA/SSc) | SSc ARA+ | Geriatric | Covid-19 |
| **Case enrolled, N** | 36 | 9 | 14 | 5 | 8 |
| **Age median (Range), years** | 84 (64-97) | 57 (38-77) | 54 (47-62) | 62 (52-88) | 51 (41-60) |
| **Gender m/f** | 19/17 | 3/6 | 4/10 | 3/2 | 5/3 |

| **DIF BMZ** | | | | | |
|---|---|---|---|---|---|
| C3+++/IgG++, C3++/IgG+ | 18 (50) | NA | NA | NA | NA |
| Firbrinogen ++ or +++ | 8 (22) | | | | |
| Negative | 3 (8) | | | | |
| Not available | 7 (19) | | | | |

| **ÎFA** | | | | | |
|---|---|---|---|---|---|
| N (% pos) | 31 (86) | | | | |
| median titer (range) | 320 (80-5120) | | | | |

| **ELISA MBL** | | | | | |
|---|---|---|---|---|---|
| BP180 U/mL mean (95% CI) | 38.3 (26.5-50) | | | | |
| N (%pos) | 26 (72) | | | | |
| BP230 U/mL mean (95% CI) | 37 (24-50) | | | | |
| N (%pos) | 23 (64) | | | | |

**Table 3. Patient characteristics. Site 2. BP= bullous pemphigoid, DH= dermatitis herpitiformis, tissue transglutaminase positive autoantibodies, EBA = Epidermolysis bullosa acquisita, collagen VII positive autoantibodies, PV= pemphigus vulgaris, desmoglin positive autoantibodies.**

| **Table 3** | **BP site 2** | **Disease Controls site 2** | | |
|---|---|---|---|---|
| | | **DH** | **EBA** | **PV** |
| **Case enrolled, N** | 26 | 53 | 6 | 2 |
| **Age median (Range), years** | 71 (29-87) | 64 (31-78) | | |
| **Gender m/f** | | | | |
| **DIF BMZ** | | | | |
| C3+++/IgG++, C3++/IgG+ | 22 (84,6) | | | |
| Firbrinogen ++ or +++ | | | | |
| Negative | 4 (15,4) | | | |
| Not available | 0 (0) | | | |

| **ÎFA** | | | | |
|---|---|---|---|---|
| N (% pos) | | | | |
| median titer (range) | | | | |

| **ELISA MBL** | | | | |
|---|---|---|---|---|
| BP180 U/mL mean (95% CI) | 113,4 (50-177) | | | |
| N (%pos) | 4 (15) | | | |
| BP230 U/mL mean (95% CI) | 36 (20-51) | | | |
| N (%pos) | 7 (27) | | | |

In order to identify the peptides with epitopes of interest based on the criteria of common reactivity by different BP serum and having highest relative intensity of their fluorescence signals, a peptide micro array (PepPerPrint^{©}microarray (https://www.pepperprint.com/), wherein the continuous epitope amino acid sequences are protected from truncation by neutral GSGSGSG linkers at the C- and N-termini has been used. The relative binding of different patient sera is measured by semi-quantification of the human IgG fluorescence intensities.

Fig. 1 illustrates the indirect immune fluorescence testing of the PepPerPrint ^{®} microarray constructed from 726 different 15-mer peptides with a peptide-peptide overlap of 14 amino acids, applied in duplicate spots (1452 spots) on to the solid support. Fig. 1A illustrates the pattern of IgG reactivities produced by representative serum samples. Fig. 1B illustrates semi-quantification of the fluorescence intensities generated by 12 bullous pemphigoid (BP) and 2 control autoimmune serum (Al) of site 1. The amino acid sequence is listed corresponding to the position of each peak in the array. The peptides of the invention originate from the amino acid sequences of BP180 non-collagenous 16a domain (NC16a) (SEQ ID NO: 11), BP180 C-terminus domain (SEQ ID NO: 12) and the BP230 C-terminus domain (SEQ ID NO: 13) (see Table 1).

The PepSlide Spotxel^{®} Microarray Image and Data Analysis Software (Sicasys Software GmbH) identified ten peptides containing epitopes of interest (SEQ ID NO:1 to 10).

Seven of these peptides were selected and prioritized for testing diagnostic performance and were synthesized in bulk with C-terminal PEG-biotin linker for conjugation to streptavidin microtiter plates.

| **origin** | | **peptide nr** | **sequence** | **Amino acid position** | **SEQ ID NO:** |
|---|---|---|---|---|---|
| BP180 | C-ter | pep1 | GGAGSLGAGGAFGEA | 1295 -1329 | 2 |
| BP180 | C-ter | pep2 | GPAGPPGHPGPPGPR | 1455-1469 | 3 |
| BP230 | C-ter | pep3 | EGLlTL TELAOSLLS | 2421 -2435 | 4 |
| **BP230** | **C-ter** | pep4 | SSHMLTDTKTGLHFN | 2387 - 2402 | 6 |
| BP230 | C-ter | pep5 | INEAIEQGTIDKALV | 2403-2416 | 8 |
| **BP180** | **C-ter** | pep6 | AFGEAAGDRGPYGTDI | 1326-1339 | 9 |
| **BP180** | **NC16** | pep7 | VDELERIRRSILPYGDSMDRIEKDR* | 491-521 | 1 |

| | | | | | |
|---|---|---|---|---|---|
| * two epitopes | | | | | |

### Example 3

Pep7 (SEQ ID NO: 1) comprises two epitopes ("multi-epitope"), which has been selected as an exemplary peptide for further tests to evaluate a diagnostic method comprising the peptides of the invention in comparison to the methods known in the art.

Firstly, the sensitivity and specificity were evaluated in a receiver operator curve (ROC) by computation of sensitivity vs. 100- specificity (Fig. 2A).

The diagnostic performance of pep7 BP180 NC16 streptavidin ELISA showed 81% sensitivity (95%, confidence interval (Cl) of 0.64 to 0.918) and 92% specificity (95%, Cl of 0.78 to 0.98) obtained with N=36 BP and N=36 controls of site 1. The ROC analysis of OD₄₅₀₋₆₂₀ values delivers the cut-off value with likelihood ratio (LR). The positive high likelihood ratio (+LR) =9.9, negative likelihood ratio (-LR) =0.21 indicate strong diagnostic accuracy. The area under the curve (AUC), which depicts the probability of a true positive diagnosed subject, is 0.937, which is considered as highly accurate, while lower AUC values are considered less accurate.

Fig. 2b illustrates the distribution of autoantibody reactivities in peptide 7 BP180 NC16a streptavidin ELISA with high statistical significance, p<0.0001, between mean OD₄₅₀₋₆₂₀ₙₘ of the thirty-six BP versus the mean OD_{450-620nm of} all thirty-six controls.

The Pasing-Bablok method comparison (Fig. 2C) shows the similarity between two different methods in linear functions, wherein the methods are identical as the linear function starts at the zero point (0,0) and has a slope of 1. Pep 7 BP180 NC16a ELISA OD₄₅₀₋₆₂₀ₙₘ values strongly correlated with the autoantibody activity (U/mL) measured by the commercial BP180 NC16a ELISA (MBL, Japan) R²=0.776, p<0.0001. Ten of 36 (28%) BP sera tested negative in the BP180 NC16a ELISA and 2 of 36 (5%) had borderline reactivity compared to 1 of 36 (2.8%) testing negative and 4 of 36 (11%) borderline in the peptide 7 BP180 NC16 ELISA.

The method comparison provides evidence that the analytical sensitivity of NC16a peptide ELISA is higher than that of the full length BP180 NC16a domain ELISA for the detection of BP autoantibodies. Thus, the native folding and secondary structure as provided in full-length epitope containing domains are not required for diagnostic methods. The use of peptides comprising the epitope without additional structural elements necessary for native folding provides a simple, hence cost-effective, improved alternative for the use in ELISA.

Fig. 3 confirms the diagnostic performance of pep7 BP180 NC16 streptavidin ELISA at 75% sensitivity (95% Cl 0.53 to 0.90) and 92 specificity (95% Cl 0.82 to 0.97) using clinical specimens of site 2 comprised of N=24 BP disease group and N=61 controls with non-BP blistering diseases.

Fig. 3b illustrates the cut-off value OD ₄₅₀₋₆₂₀ₙₘ > 0.49 obtained using clinical specimens of site 2 is approximately 3-fold higher than the cut-off value OD₄₅₀₋₆₂₀ₙₘ >0.168 obtained using the clinical specimens of site 1. Upon comparing the test results of BP and controls of site 1 on two inter-day runs, the second run on the same microtiter plates as the clinical specimens of site 2, we found the BP site 1, run 1 values OD ₄₅₀₋₆₂₀ₙₘ mean 1.25 (95% CI 0.87 to 1.63) were similar to the run 2 values OD₄₅₀₋₆₂₀ₙₘ mean 1.38 (95% Cl 1.0 to 1.7). However, the Al controls run 1 OD₄₅₀₋₆₂₀ₙₘ mean 0.05 (95% CI 0.037 to 0.06) were 8-fold lower than the run 2 OD₄₅₀₋₆₂₀ₙₘ mean 0.40 (95% Cl 0.37 to 0.43) and the COVID-19 run 1 OD ₄₅₀₋₆₂₀ₙₘ mean 0.07 (95% CI 0.62 to 0.84) 6-fold lower than the COVID-19 run 2 OD ₄₅₀₋₆₂₀ₙₘ mean 0.44 (95% CI 0.40 to 0.48).

The demonstrably increased background reactivity of control specimens in the second run on streptavidin microtiter plates (Biomat) compared to the first run on streptavidin microtiter plates (Greiner Bio-one) are likely attributed to technical differences in the peptide coating and inter-run variation.

Fig. 2 and Fig. 3 illustrate outcomes of pep7 BP180 NC16 streptavidin ELISA diagnostic performance that were not confounded by the inter-day run technical issues. The clinical specimens collected at two independent sites gave similar sensitivity and specificity with ROC analysis of high statistical significance, AUC 0.94, p<0.001 and 0.87, p<0.001.

In fact, the findings in Fig. 3 reinforce the robust performance of the pep7 BP180 NC16 streptavidin ELISA in the diagnosis of BP at site 2.

Fig. 3a/3b illustrate that the pep7 BP180 NC16 ELISA clearly differentiates between autoantibodies of BP patients which target the distinct immunodominant epitopes of BP180 NC16a on the basolateral membrane zone versus the autoantibodies of other autoimmune blistering diseases which target intra-epidermal autoantigens; keratinocyte-associated tissue transglutaminases of DH (dermatitis herpitiformis), collagen VII of EBA (Epidermolysis bullosa acquisita) and desmogleins of PV (pemphigus vulgaris) patients.

Fig. 2b shows positive pep7 BP180 NC16 reactivity detected in five ARA+ SSc autoimmune controls; OD₄₅₀₋₆₂₀ₙₘ mean 0.24 (95% CI 0.21 to 0.27) and one geriatric control with stage 3 Parkinson's disease; OD₄₅₀₋₆₂₀ₙₘ=0.26. These values reside within 1SD above the 0.168 cut-off and are unsurprising. Both anti-RNA polymerase [40] and BP180 autoantibodies [26] have strong associations with malignancy and neurological inflammation [6-9]. Pre-clinical BP180 autoantibodies have reported 1 to 4 percent prevalence in normal healthy individuals [41]

Fig. 3b shows that the pep7 BP180 NC16 ELISA detects low levels of autoantibodies in DH patients; 2 of 61 (3%) gave OD₄₅₀₋₆₂₀ₙₘ >0,49 cut off and borderline reactivity 5 of 61 (8%). Although we have not directly tested these sera in the BP180 NC16 ELISA, a previous publication reported DH patients, 3 of 46 (6.5%) having positive reactivity in MBL BP180 NC16 ELISA [42].

### Example 4

Fig. 4 illustrates a consistent trend of greater reactivity of BP patient sera than that of control sera in both the peptide 6 (pep6) BP180 C-terminus ELISA and in the peptide 4 (pep4) BP230 C-terminus ELISA.

Fig. 4a illustrates statistical significance between mean OD₄₅₀₋₆₂₀ₙₘ values of BP sera site 1 versus mean OD₄₅₀₋₆₂₀ₙₘ of autoimmune sera site 1 in the peptide 6 BP180 C-terminus ELISA.

Fig. 4a/4b filled symbols represent six BP patients; 4 of 36 (11%) site 1 and 2 of 24 (8%) site 2, that test positive in the peptide 4 BP230 ELISA and test negative in the peptide 7 BP180 NC16a ELISA. Pep4 has near 100 % specificity (higher than pep7). The finding has relevance for the use of BP230 peptide epitopes in closing the diagnostic gap in BP180 seronegative BP patients. Fig. 4b illustrates DH patients 7 of 53 (13%) that test positive in the peptide 6 BP180 C-terminus ELISA of and 10 of 53 (19%) that test positive in the peptide 4 BP230 C-terminus ELISA. The finding underscores the potential of peptide epitopes to detect formation of anti-desmosome autoantibodies in a subset of anti-tTg (tissue transglutaminase) positive DH patients. Further longitudinal studies are required to determine the clinical relevance of these reactivities in the DH skin lesion immunopathology.

The sample derived from a subject is considered in the present invention to indicate the presence of bullous pemphigoid, when the OD₄₅₀₋₆₂₀ nm value of Pep7 NC16a ELISA and/or the OD 450-620 nm value of Pep4 BP230 C-terminus ELISA is above the cut-off determined from ROC analysis.

### Example 5

Fig. 5 illustrates the capacity of peptide 7 BP180 NC16a to block binding of BP patient autoantibodies to the BP180 NC16a protein in the BP180 NC16a MBL ELISA. The incubation of patient sera with peptide concentrations in the range of >3µg to 100µg/mL resulted in 20 to 80 percent decrease in the autoantibody binding activity (U/mL), depending on the individual BP patient. These results confirm that the immunodominant NC16a epitope displayed on the Pep7 25mer peptide competes with native NC16a domain of the recombinant protein and for certain BP patients effectively blocks the binding activity of autoantibodies measured by commercial ELISA.

Fig. 6 illustrates that the pre-incubation of peptide 7 BP180 NC16a with patient sera at concentrations between 10 and 100 µg/mL, enhanced the indirect immune fluorescence on the epidermal basement membrane of monkey esophagus tissue preparations relative to the control sera alone without peptide. The finding indicates that the peptide rather than neutralizing the autoantibody binding in the tissue, facilitates the accumulation of patient IgG in the tissue as autoantibody-peptide complex.

### References

1. 2022 Deotto ML, Spiller A, Sernicola A and Alaibac M. Bullous pemphigoid: An immune disorder related to aging (Review). Exp Ther Med; 23: 50-58.
2. 2022 Zhou T and Geng S. Emerging biomarkers and therapeutic strategies for refractory bullous pemphigoid Front Immunol 24:12:718073. https://doi:10.3389/fimmu.2021.718073.
3. 2019 Stevens NE, Cowin AJ and Kopeck Z. Skin Barrier and Autoimmunity-Mechanisms and Novel Therapeutic Approaches for Autoimmune Blistering Diseases of the Skin. Front Immunol; 10: 10.3389 https://doi:10.3389/fimmu.2019.01089.
4. 2021 Tsiogka A, Bauer JW and Patsatsi A. Bullous Pemphigoid Associated with Anti-programmed Cell Death Protein 1 and Anti-programmed Cell Death Ligand 1 Therapy: A Review of the Literature. Acta Derm Venereol 101: adv00377. https://doi.org/10.2340/00015555-3740.
5. 2019 Douros A, Rouette J, Yin OHH Yu, Filion KB and Azoulay L. Dipeptidyl peptidase 4 inhibitors and the risk of bullous pemphigoid among patients with type 2 diabetes. Diabetes Care; 42:1496-1503. https://doi.org/10.2337/dc19-0409.
6. 2017 Kokkonen N, Herukka SK, Huilaja L, Kokki M, Kovisto AM, Hartikainen P, Remes AM and Tasanen K. Increased levels of bullous pemphigoid BP180 autoantibody are associated with more severe dementia in Alzheimer's disease. J Invest Dermatol; 137:71-76. https://doi.org/10.1016/j.jid.2016.09.010.
7. 2019 Messingham KN, Miller AD, Narayanan NS, Connell SJ and Fairley JA. Demographics and Autoantibody Profiles of Pemphigoid Patients with Underlying Neurologic Diseases. J Invest Dermatol 139:1860e1866; https://doi.org/10.1016/j.jid.2019.01.034.
8. 2022 Opelka B, Schmidt E and Goletz S. Type XVII collagen: Relevance of distinct epitopes, complement-independent effects, and association with neurological disorders in pemphigoid disorders. Frontiers Immunol 13:1-11 https://doi.org/10.3389/fimmu.2022.948108.
9. 2021 Ständer S, Hammers CM. Coexistence of bullous pemphigoid with neuropsychiatric comorbidities is associated with anti-BP230 seropositivity. J Eur Acad Dermatol Venereol; 35: 1919-1920 https://doi:10.1111/jdv.17304.
10. 2019 Genovese G, Di Zenzo G, Cozzani E, Berti E, Cugno M and Marzano AV. New insights into the pathogenesis of bullous pemphigoid: 2019 update. Front Immunol; 10: 1506. https://doi.org/10.3389/fimmu.2019.01506.
11. 2014 Antiga E, Quaglinio P, Volpi W, Pierini I, Del Bianco E, Bianchi B, Novelli M, Savoia, MG, Bernengo P, Fabbri P and Caproni M. Regulatory T cells in skin lesions and blood of patients with bullous pemphigoid. J Eur Acad Dermatol Venerol; 14:222-230. https://doi.org/10.1111/idv.12091.
12. 1997 Ziljkens D, Rose P. et al. Tight clustering of extracellular BP180 epitopes recognized by bullous pemphigoid autoantibodies. J Invest Dermatol; 109:573-579.
13. 1996 Matsumura K, et al.: The majority of bullous pemphigoid and herpes gestationis serum samples react with NC16a domain of the 180kD bullous pemphigoid antigen. Arch Dermatol Res 288:507-509.
14. 2016 Wada M, Nishie W et al. Epitope-dependent pathogenicity of antibodies directed against targeting a major bullous pemphigoid autoantigen Collagen XVII /BP180. J Invest Dermatol; 136:938-946. https://doi.org/10.1016/j.jid.2015.11.030.
15. 2004 DiZenzo G, Grosso F et al. Characterization of the anti-BP180 autoantibody reactivity profile and epitope mapping in bullous pemphigoid patients. J Invest Dermatol 122:103-110.
16. 2018 Zhang Y, Hwang BJ et al. BP180 dysfunction triggers spontaneous skin inflammation in mice. Proc Natl Acad Sci; 119:6434-6439. https://doi.org/10.1073/pnas.1721805115.
17. 2010 Wang G, Ujiie H et al. Blockade of autoantibody-initiated tissue damage by using recombinant Fab antibody fragments against pathogenic autoantigen. Am J Pathol; 176: 914-925.
18. 2013 Fairley JA, Bream M et al. Missing the target: Characterization of bullous pemphigoid patients who are negative using the BP180 enzyme-linked immunosorbant assay. J Am Acad Dermatol; 68: 395-403. https://doi:10.1016/i.iaad.2012.09.012.
19. 2001 Hamada T et al. Role of BP230 autoantibodies in bullous pemphigoid. Experimental Dermatology; 10:256-263.
20. 2023 Hideyuki U. What's new in the pathogeneses and triggering factors of bullous pemphigoid. J Dermatol; 50: 140-149.
21. 2022 Cole C, Borradori L and Amber KT. Deciphering the contribution of BP230 autoantibodies in bullous pemphigoid. Pemphigoid. MDPI Antibodies; 11: 44-55. https://doi.org/10.3390/antib11030044.
22. 2022 Anti-BP230 Only Bullous Pemphigoid Constitutes a Distinct Disease Subgroup with Characteristic Serological and Clinical Features. J Invest Dermatol; https://doi:10.1016/j.jid.2022.05.1084.
23. 2019 Ujiie H, Yoshimoto N et al. Immune Reaction to Type XVII Collagen Induces Intramolecular and Intermolecular Epitope Spreading in Experimental Bullous Pemphigoid Models. Front Immunol; 10:3389 https://doi:10.3389/fimmu.2019.01410.
24. 2020 Seo T, Ujiie H, Ujiie I, Iwata H, Shimizu H. Epitope spreading possibly from BP230 to the NC16A domain of BP180 preceding disease progression in bullous pemphigoid. J Dermatol ;47:e255-7.
25. 2021 Tsutusui K, Machida H et al. Mapping the molecular and structural specialization of the skin basement membrane for inter-tissue interactions. Nat Commun; 12:2577 https://doi.org/10.1038/s41467-012-2281-y.
26. 2023 Crespo-Bravo M, Thorlacius-Ussing J et al. Levels of type XVII collagen (BP180) ectodomain are elevated in circulation from patients with multiple cancer types and is prognostic for patients with metastatic colorectal cancer. BMC Cancer 23:949 https://doi:10.1186/s12885-023-11470-5.
27. 1989 Wiegand DA and Clements MK. Direct immunofluorescence in bullous pemphigoid: effects of extent and location of lesions. J Am Acad Dermatol: 20:437-440.
28. 1991 Sarret Y, Hall R, Cobo M, Thivolet J, Patton DL, Woodley DT. Salt-split human skin substrate for the immunofluorescent screening of serum from patients with cicatricial pemphigoid and a new method of immunoprecipitation with IgA antibodies. J Am Acad Dermatol; 24: 952-958.
29. 2008 Barnadas MA, Rubiales MV, Gonzalez MJ, Puig L, Pilar G, Baselga E, Pujol R, Alomar A, Gelpi C. Enzyme-linked immunosorbent assay (ELISA) and indirect immunofluorescence testing in a bullous pemphigoid and pemphigoid gestationis. International J Dermatol 47:1245-1249.
30. 2021 Simpson K, Scardamaglia L, Kok Y et al. Comparison of the EUROIMMUN Dermatology Profile ELISA to the novel BIOCHIP Mosaic 7 for the diagnosis of immunobullous skin disease, Australas J Dermatol; 62:314-322 https://doi.org/10.1111/aid.13611.
31. 1996 Matsumura K, et al.: The majority of bullous pemphigoid and herpes gestationis serum samples react with NC16a domain of the 180kD bullous pemphigoid antigen. Arch Dermatol Res; 288: 507-509. https://doi.10.1007/BF02505245.
32. 2006 Yoshida M, Hamada T, Amagai M, et al.: Enzyme-linked immunosorbent assay using bacterial recombinant proteins of human BP230 as a diagnostic tool for bullous pemphigoid. J Dermatol Sci; 41:21-30. https://doi.10.1016/j.jdermsci.2005.11.002
33. 2012 Blöcker IM, Dähnrich C, Probst C, Komorowski L, Saschenbrecker S, Schlumberger W, Stöcker W, Zillikens D, Schmidt E. (EUROIMMUN AG). Epitope mapping of BP230 leading to a novel enzyme-linked immunosorbent assay for autoantibodies in bullous pemphigoid. Br J Dermatol 166: 964-970.
34. 2004 Thoma-Uszynski S, Uter W, Schwietzke S, Hofmann SC, Hunziker T, et. Al.: BP230- and BP180-specific autoantibodies in bullous pemphigoid. J Invest Dermatol. 122:1413-22.
35. 2018. Clinical evaluation of a multiparametric ELISA as a rapid tool for routinely diagnosing IgG-mediated autoimmune blistering dermatoses in ethnic Slavs. J Clin Lab Anal 32: 2236 https://doi.org.10.1002/jcla.22336.
36. 2021 Goletz S, Giurdanella F, Holtsche MM et al. Comparison of Two Diagnostic Assays for Anti-Laminin 332 Mucous Membrane Pemphigoid. Front Immunol; 12: 773720. https://doi.org/10.3389/fimmu.2021.773720.
37. 2019 Hammers MC, Siegel DL, Stanley JR .PCT USA2019/054751, WO 2020/072937 A1 Antibodies against membrane zone adhesion proteins BP180 and BP230.
38. 1999 Nie Z, Garrod DR, Chan LS, Hashimoto T. Complementary peptides against the major epitope in the NC16A domain of BP180 show no specificity as vaccines to bullous pemphigoid. J Dermatol Sci; 21:157-64. https://doi.org/10.1016/S0923-1811(99)00033-X.
39. 2000 Xu L, O'Toole EA, Olivrry T et al. Molecular cloning of canine bullous pemphigoid antigen 2 cDNA and immunomapping of NC16A domain by canine bullous pemphigoid autoantibodies . Biochem et Biophys Acta; 1500: 97-107. https://doi.org/10.1016/S0925-4439(99)00092-7.
40. 2014 Moinzadeh P, Fonseca C, Hellmich M, Shah AA, Chighizola C, Denton CP and Ong VH. Association of anti-RNA polymerase III autoantibodies and cancer in scleroderma. Arthritis Res and Ther 16:R53 http://arthritis-research.com/content/16/1/R53.
41. 2022 Mai Y, Izumi K, Mai S and Ujiie H. The significance of preclinical anti-BP180 autoantibodies. Front Immunol 13: 96340. https://doi.org/10.3389/fimmu.2022.963401.
42. Natnyki A, Tuusa J, Hervonen K et al. Autoantibodies against the immunodominant bullous pemphigoid epitopes are rare in patients with dermatitis herpetiformis and coeliac disease. Front Immunol 11:575805. https://doi.org.10.3389/fimmu.2020.575805.
43. Di Zenzo G, Grosso F, Terracina M, Mariotti F, De Pità O, Waribe K, Mastragiacomo A, Sera F, Borradori L, and G. Zambruno, 2004, Chracteriziation of the Anti-BP180 Autoantibody Reactivity Profile and Epitope Mapping in Bullous Pemphigoid Patients. J Invest Dermatol. 122:103-110.
44. Okuzumi, A., Hatano, T., Matsumoto, G. et al. Propagative α-synuclein seeds as serum biomarkers for synucleinopathies. Nat Med 29, 1448-1455 (2023). https://doi.org/10.1038/s41591-023-02358-9.
45. Palermo, G.; Giannoni, S.; Bellini, G.; Siciliano, G.; Ceravolo, R. Dopamine Transporter Imaging, Current Status of a Potential Biomarker: A Comprehensive Review. Int. J. Mol. Sci. 2021, 22, 11234. https://doi.org/10.3390/ijms222011234.

## Claims

1. A diagnostic composition comprising at least one peptide comprising an amino acid sequence selected from the group consisting of SEQ ID NO: 1 (VDELERIRRSILPYGDSMDRIEKDR), SEQ ID NO: 2 (GGAGSLGAGGAFGEA), SEQ ID NO: 3 (GPAGPPGHPGPPGPR), SEQ ID NO: 4 (EGLITLTELADSLLS), SEQ ID NO: 5 (EFQYLTGGLIEPQVH), SEQ ID NO: 6 (SSHMLTDTKTGLHFN), SEQ ID NO: 7 (FIPGPPGPPGPPGPR), SEQ ID NO: 8 (INEAIEQGTIDKALV), SEQ ID NO: 9 (AFGEAAGDRGPYGTDI) and SEQ ID NO: 10 (LITLTELADSLLSRL).

2. The diagnostic composition of claim 1, wherein the composition comprises at least two, three, four or more of said peptides.

3. The diagnostic composition of claims 1 or 2, wherein the peptide is that of SEQ ID NO: 1 (VDELERIRRSILPYGDSMDRIEKDR).

4. The diagnostic composition of any of the preceding claims, wherein the peptides comprise spacer and/or linker sequences for immobilization.

5. The diagnostic composition of claim 4, wherein the spacer and/or linker is a PEG-biotin linker.

6. The diagnostic composition of any one of claims 1 to 5, wherein the peptides are immobilized on a solid support, preferably a streptavidin coated microtiter plate.

7. The diagnostic composition of any one of claims 1 to 5, wherein the peptides are joined by cyclization.

8. The diagnostic composition of any one of claims 1 to 7 for evaluating the presence or absence of a disease selected from the group of bullous pemphigoid, Parkinson's disease, and a neurological disorder.

9. A kit comprising at least one peptide identified in any of the preceding claims.

10. An in vitro method for detecting the presence or absence of a disease selected from the group of bullous pemphigoid, Parkinson's disease, and a neurological disorder comprising testing the binding of at least one peptide as identified in any of the preceding claims for binding to autoantibodies obtained from a subject suspected of suffering from one of said diseases.

11. The *in vitro* method according to claim 10 which is an ELISA.

12. A pharmaceutical composition comprising at least one peptide as identified in any of the preceding claims.

13. The at least one peptide as identified in any of the preceding claims for use in the treatment of a disease selected from the group of bullous pemphigoid, Parkinson's disease, and a neurological disorder.
